# EUROPEAN PATENT APPLICATION

(11) **EP 4 835 049 A1**
(43) Date of publication of application: **04.11.2026**
(21) Application number: 24913251.5
(22) Date of filing: 31.07.2024
(51) Int. Cl.: C12N 1/20, C08J 11/10, B09B 3/60, B09B 101/75, C12R 1/01, C12R 1/22

(54) **NOVEL MICROORGANISM WITH PLASTIC DEGRADATION ACTIVITY AND USE THEREOF**

(30) Priority: 26.12.2023 KR 20230191102
(71) Applicant: Repla Inc., Gyeongsan-si, Gyeongsangbuk-do 38541 (KR)
(72) Inventor: KIM, Hong Rae, Suwon-si Gyeonggi-do 16678 (KR); SHIN, Hye Young, Yongin-si Gyeonggi-do 17176 (KR); SUH, Dong Eun, Yongin-si Gyeonggi-do 17103 (KR)
(74) Representative: Maiwald GmbH
(86) International application number: PCT/KR2024/011261
(87) International publication number: WO 2025/143410

(57) **Abstract**

The present invention relates to a novel microorganism with plastic degradation activity. The microorganism of the present invention can degrade plastics synthesized from petroleum-based compounds such as polyethylene (PE), polystyrene (PS), polypropylene (PP), polyethylene terephthalate (PET), polyurethane (PU), and polyvinyl chloride (PVC) and convert same into low-molecular-weight materials, and is excellent in degrading plastics or plastic metabolic intermediates, and thus can be used in a pretreatment process for recycling plastics.

## Description

### [Technical field]

The present invention relates to a novel microorganism capable of degrading various plastics and a method for degrading plastics using the same.

### [Background Art]

In modern society, plastic has become an indispensable industrial material. Plastic materials have been widely used in various industries for the past 50 years due to their high strength, elasticity, light weight, low production cost, and ease of use. Plastic production increased significantly from 234 million tons in 2000 to 460 million tons in 2019. In addition, the amount of plastic waste generated during the same period more than doubled from 156 million tons to 353 million tons. According to a UN report, plastic production is expected to reach 800 million tons by 2035 and 1.6 billion tons by 2050, of which more than two-thirds (76%) will be treated as waste; the disposal of this waste will rely on recycling for only 9%, incineration for 12%, and landfill for 79%.

Among methods for treating plastic waste, landfilling is the most economical and common, but it requires extensive landfill sites; meanwhile, incineration at high temperatures can reduce waste accumulation, but it generates toxic compounds such as dioxins and carbon monoxide during the combustion process, thereby causing another form of environmental pollution. Physical and chemical recycling are environmentally friendly methods, but they are used very restrictively due to limitations such as the low efficiency of the separation/sorting process and the economic utility of recycled plastics.

In addition, environmental pollution caused by plastic influx not only threatens ecosystems and human health but also incurs additional costs for waste management and pollution remediation; thus, improvements and research on plastic waste treatment methods are actively being conducted.

As an example of research on plastic-degrading microorganisms, Korean Patent Registration No. 10-0350928 discloses a novel microorganism, *Klebsiella pneumoniae* CJ-PVA a (accession number KFCC-11126), which exhibits good growth under aerobic conditions and an improved polyvinyl alcohol-degrading ability, and a method of treating wastewater containing polyvinyl alcohol using the same (Patent Document 1). In addition, Korean Patent Registration No. 10-0513931 discloses *Microbacterium barkeri* LC (accession number KCCM 10507) and a method of biologically degrading polyvinyl alcohol using the same (Patent Document 2).

However, the aforementioned plastic-degrading microorganisms have a disadvantage in that the technology is limited to the degradation of polyvinyl alcohol, which has high surface activity and biodegradability, making it difficult to apply to the degradation of plastics synthesized from other petrochemicals, and thus difficult to utilize as microbial species or combinations for degrading various types of plastics.

### [Detailed Description of Invention]

### [Technical Problem]

Under the circumstances described above, the inventors have made diligent research efforts to find novel microorganisms capable of degrading various plastics.

As a result, the present invention was completed by isolating a novel microorganism capable of degrading various plastics.

An object of the present invention is to provide a *Rhodococcus yunnanensis* REPLA5 strain having plastic-degrading activity, deposited under accession number KACC 81286BP.

Another object of the present invention is to provide a method of degrading a plastic, which includes culturing a plastic with the *Rhodococcus yunnanensis* REPLA5 strain deposited under accession number KACC 81286BP, a culture broth of the strain, a cell lysate of the strain, or a plastic-degrading enzyme derived from the strain.

Still another object of the present invention is to provide a composition for plastic degradation, which includes the *Rhodococcus yunnanensis* REPLA5 strain deposited under accession number KACC 81286BP, a culture broth of the strain, a cell lysate of the strain, or a plastic-degrading enzyme derived from the strain.

Yet another object of the present invention is to provide a method of degrading a plastic metabolic intermediate, which includes culturing a plastic metabolic intermediate with the *Rhodococcus yunnanensis* REPLA5 strain deposited under accession number KACC 81286BP, a culture broth of the strain, a cell lysate of the strain, or a plastic-degrading enzyme derived from the strain.

Yet another object of the present invention is to provide a composition for metabolic intermediate degradation, which includes a *Rhodococcus yunnanensis* REPLA5 strain deposited under accession number KACC 81286BP, a culture broth of the strain, a cell lysate of the strain, or a plastic-degrading enzyme derived from the strain.

Yet another object of the present invention is to provide a composition for promoting plastic oxidation, which includes a *Rhodococcus yunnanensis* REPLA5 strain deposited under accession number KACC 81286BP, a culture broth of the strain, a cell lysate of the strain, or a plastic-degrading enzyme derived from the strain.

### [Technical Solution]

Hereinafter, the present invention will be described in more detail.

One aspect of the present invention is a *Rhodococcus yunnanensis* REPLA5 strain having plastic-degrading activity, deposited under accession number KACC 81286BP.

The present inventors isolated a strain having a plastic-degrading ability from waste polyethylene vinyl and performed 16S rRNA sequencing (SEQ ID NO: 1) to identify a pure single strain through subculturing, confirming that the obtained strain belongs to the genus *Rhodococcus*. The isolated microorganism was named *Rhodococcus yunnanensis* REPLA5 and deposited with the Korean Agricultural Culture Collection (KACC) on December 7, 2023, receiving accession number KACC 81286BP.

In the present invention, the "genus *Rhodococcus*" is a non-spore-forming aerobic bacterium classified as a *Nocardia*-type actinomycete containing mycolate. The genus *Rhodococcus* consists of genetically and physiologically diverse bacteria, but they can be isolated from soil or seawater and utilized, perform various enzymatic functions, and have unique cell wall structure. The cell wall of *Rhodococcus* contains mycolic acid, as in the genera *Mycobacteria*, *Nocardia*, and *Corynebacteria*. The long-chain lipid mycolic acid in the cell envelope facilitates the intracellular absorption of hydrophobic substrates. In addition, the ability to metabolize hydrophobic substrates enables the production of surfactants. The compound generated in this way attaches cells to the hydrophobic phase, increasing interfacial tension and dispersing the hydrophobic substrate. Since substrates present in the proliferation medium alter the fatty acid composition of biomembrane lipids and thus change the fluidity of the cell envelope, changes in the fatty acid composition play an important role in the resistance of *Rhodococcus* cells to toxic chemicals. *Rhodococcus* strains form a biofilm on a carrier, increasing resistance to toxic chemicals. The genus *Rhodococcus* has resistance to xenotoxicity and degradability and is present in areas contaminated with aromatic compounds. *Rhodococcus* degrades benzene, toluene, xylene, biphenyl, polycyclic aromatics, phenol, PCBs, aniline, ethers, pesticides, and the like, and also functions as a desulfurizing bacterium.

In the present invention, the plastic may be one or more selected from the group consisting of polyethylene terephthalate (PET), polyvinyl chloride (PVC), polystyrene (PS), polypropylene (PP), polyurethane (PU), and polyethylene (PE), but is not limited thereto.

The polyethylene terephthalate is a saturated polyester resin with a relatively high density and is a crystalline or amorphous thermoplastic plastic. Amorphous polyethylene terephthalate has high transparency but exhibits low tensile strength and low sliding properties and thus is mainly used for bottles or packaging applications. On the other hand, crystalline polyethylene terephthalate has high hardness, rigidity, and strength, along with excellent sliding properties and abrasion resistance and is therefore used by Ensinger as a semi-finished product for machining.

The polyvinyl chloride is similar to polypropylene but has a form in which one hydrogen is substituted for one chlorine. In the case of polyvinyl chloride, studies have primarily focused on the fact that the plasticizers included during production (e.g., bis-2-ethylhexyl phthalate, dioctyl adipate, and the like), rather than the main carbon chain of polyvinyl chloride, are partially degraded by bacteria and fungi collected from various environments and converted into low-molecular-weight materials.

Although the above polystyrene is structurally similar to polyethylene terephthalate (PET), high-density polyethylene HDPE, and low-density polyethylene HDPE (LDPE), it has higher hydrophobicity due to the structural characteristics of its monomers, resulting in minimal hydrolysis; it is known to have the slowest degradation rate in the natural environment among generally classified plastics. Polystyrene, which is a type of thermoplastic, is lightweight, tasteless, and odorless, and thus is used in household goods, toys, electrical insulators, radio and television cases, packaging materials, and the like. Polypropylene is produced by polymerizing propylene obtained from petroleum and is widely used for items such as bottles and containers.

The polypropylene is a polymer obtained by polymerizing propylene via Ziegler-Natta polymerization or metallocene catalyst polymerization. Polypropylene consists of methyl groups (CH₃) attached to every other carbon in the polyethylene molecular chain and has a structure with regularly arranged short branches. Polypropylene has a relatively simple molecular structure connected by carbon bonds, but similar to polystyrene, it is difficult to hydrolyze due to the methyl groups included in each bond.

The polyurethane is based on a urethane bond (-NHCOO-) structure formed by the polymerization reaction of isocyanate and polyol, and various diols are added as chain extenders. Basically, urethane bonds themselves are known to be vulnerable to microbial attack, but since polyester or polyether bonds present within polyols such as polycaprolactone (PCL) or polyethylene glycol account for the vast majority of the polymer structure, the biodegradation of urethane is primarily determined by the type and extent of these bonds. Polyester urethane can basically be degraded relatively more easily than polyether urethane by hydrolases such as esterases, and numerous fungi and bacteria are known to degrade it.

The polyethylene is produced by the polymerization reaction of ethylene and is composed only of carbon-hydrogen chains, making it very stable and known not to degrade for decades after landfilling, just like other plastics. In the degradation process of polyethylene, functional groups such as a hydroxyl group (-OH) and a carbonyl group (C=O) are generated by oxidation, and carbon-carbon bonds are cleaved to produce an intermediate material (fatty acid) having a relatively low molecular weight. Polyethylene is a thermoplastic plastic that is lightweight and flexible, and is a general-purpose plastic used in everything from industrial materials to everyday goods. HDPE has high impact resistance and good cold resistance and thus is mainly used to manufacture shopping bags or pipes. LDPE has branches in its polymer chain, resulting in a lower density than that of linear HDPE, and exhibits excellent flexibility, allowing for easy processing.

As used herein, the term "plastic-degrading activity" refers to an activity by which plastics and plastic metabolic intermediates can be degraded.

As used herein, the term "plastic degradation" refers to degrading a polymeric material constituting a plastic into low-molecular-weight intermediate materials or intermediates that can be metabolized through a metabolic pathway of a microorganism. In order to metabolize plastic having a high-molecular-weight structure, degradation into monomer units with a low molecular weight (Mw ~500) must occur, and the monomers are then utilized for microbial metabolism in various forms. Among various monomers, hydrocarbon structural materials ([CₙHₙ]ₙ) are primarily metabolized via the fatty acid degradation pathway (β-oxidation pathway), and metabolism can occur stepwise in the order of alkanes, alcohols, aldehydes, and fatty acids.

According to an embodiment of the present invention, it was confirmed that the *Rhodococcus yunnanensis* REPLA5 strain can degrade various types of plastics by degrading polyethylene at a degradation rate of about 3.04%, polystyrene at about 15.39%, polypropylene at about 5.0%, polyethylene terephthalate at about 7.82%, and polyurethane at 3.29% (Example 3-1).

Another aspect of the present invention is a method for degrading a plastic, which includes the step of culturing a plastic with the *Rhodococcus yunnanensis* REPLA5 strain deposited under accession number KACC 81286BP, the strain culture medium, the strain lysate, or a strain-derived plastic-degrading enzyme.

In the present invention, the plastic may be one or more selected from the group consisting of polyethylene terephthalate (PET), polyvinyl chloride (PVC), polystyrene (PS), polypropylene (PP), polyurethane (PU), and polyethylene (PE), but is not limited thereto.

In the present invention, culture may be performed by inoculating the *Rhodococcus yunnanensis* REPLA5 strain into a medium having a pH of 5 to 9, 5 to 8, 5 to 7, 6 to 9, 6 to 8, or 6 to 7, but is not limited thereto.

When the strain is grown at a pH outside the range of pH 5 to 9, the strain may not be cultured well.

In the present invention, the culture may be performed for 7 to 60 days at a temperature of 10 to 40 °C after inoculating the *Rhodococcus yunnanensis* REPLA5 strain into a medium including a plastic as a carbon source, and preferably for 10 to 60 days at 25 to 40 °C, but is not limited thereto.

When cultured at a temperature higher or lower than the above culture temperature, the strain may not be cultured well. In addition, when culture is performed for a shorter time than the above culture time, the culture may end before sufficient plastic-degrading activity appears, and when culture is performed for a longer time than the above culture time, the plastic degradation efficiency may be low relative to the culture time.

In the present invention, the culture medium composition, culture temperature, and culture time may vary depending on the type of plastic to be degraded, and when the *Rhodococcus yunnanensis* REPLA5 strain is cultured together with plastic or plastic metabolic intermediates under conditions where these conditions are combined as process parameters, a specific plastic can be degraded relatively largely or all plastics can be degraded into reusable low-molecular-weight materials.

The plastic added to the above medium may be in the form of finely broken flakes, a powder, or a thin film to increase contact with the *Rhodococcus yunnanensis* REPLA5 strain, a culture broth of the strain, a cell lysate of the strain, or a plastic-degrading enzyme derived from the strain.

According to an embodiment of the present invention, when *Rhodococcus yunnanensis* REPLA5 and a plastic powder (LDPE) were supplied as carbon sources, an increase in oxygen atoms on the plastic surface and the formation of a C-O bond and a carbonyl group (C=O) were confirmed. In addition, it was confirmed that the hydroxyl group (OH) and the carbonyl group (C=O) were formed in the chemical functional groups on the plastic surface. In addition, considering the appearance of the C-O bond confirmed in the XPS analysis, the hydroxyl group (OH) observed in the FT-IR analysis can be considered to be bound to the plastic in the form of C-OH, and it was confirmed that the *Rhodococcus yunnanensis* REPLA5 can oxidize plastic (Example 3-2).

A method of degrading a plastic according to the present invention may be applied as a pretreatment process for plastic recycling.

Another aspect of the present invention is a composition for plastic degradation, which includes the *Rhodococcus yunnanensis* REPLA5 strain deposited under accession number KACC 81286BP, a culture broth of the strain, a cell lysate of the strain, or a plastic-degrading enzyme derived from the strain.

In the present invention, since the *Rhodococcus yunnanensis* REPLA5 strain uses plastic as a carbon source, the strain itself, a culture broth of the strain, a lysate of the strain, or a plastic-degrading enzyme derived from the strain can be used for plastic degradation.

In the present invention, the plastic may be one or more selected from the group consisting of polyethylene terephthalate (PET), polyvinyl chloride (PVC), polystyrene (PS), polypropylene (PP), polyurethane (PU), and polyethylene (PE), but is not limited thereto.

Yet another aspect of the present invention is a method of degrading a plastic metabolic intermediate, which includes culturing a plastic metabolic intermediate with a *Rhodococcus yunnanensis* REPLA5 strain deposited under accession number KACC 81286BP, a culture broth of the strain, a cell lysate of the strain, or a plastic-degrading enzyme derived from the strain.

In the present invention, the plastic metabolic intermediate may be one or more selected from the group consisting of an alkane, an alcohol, an aldehyde, and a fatty acid, but is not limited thereto.

Yet another aspect of the present invention is a composition for metabolic intermediate degradation, which includes a *Rhodococcus yunnanensis* REPLA5 strain deposited under accession number KACC 81286BP, a culture broth of the strain, a cell lysate of the strain, or a plastic-degrading enzyme derived from the strain.

In the present invention, since the *Rhodococcus yunnanensis* REPLA5 strain uses plastic as a carbon source, the strain itself, the strain culture medium, the strain lysate, or the plastic-degrading enzyme derived from the strain can be used for the degradation of plastic metabolic intermediates.

Yet another aspect of the present invention is a composition for promoting plastic oxidation, which includes a *Rhodococcus yunnanensis* REPLA5 strain deposited under accession number KACC 81286BP, a culture broth of the strain, a cell lysate of the strain, or a plastic-degrading enzyme derived from the strain.

In the present invention, the composition for promoting plastic oxidation can promote catalytic action by oxidation in relation to the degradation of plastic, which is a polymer material, and the catalytic action can increase the hydrophilicity of the hydrophobic plastic surface to promote the formation of a microbial hydrophilic biofilm and depolymerization reaction.

According to an embodiment of the present invention, *Rhodococcus yunnanensis* REPLA5 corroded the plastic surface by biodegradation and formed a microbial biofilm of the REPLA5 strain on the plastic surface (Examples 2 and 3).

### [Advantageous Effects]

The present invention relates to a novel microorganism having plastic-degrading activity. The microorganism of the present invention can decompose plastics synthesized from petrochemicals such as polyethylene terephthalate (PET), polyvinyl chloride (PVC), polystyrene (PS), polypropylene (PP), polyurethane (PU), and polyethylene (PE) into low-molecular-weight materials, and is excellent at degrading plastics or plastic metabolic intermediates, and thus it can be used in a pretreatment process for plastic recycling.

### [Description of Drawings]

FIG. 1 shows the results of confirming the number of viable cells after culturing *Rhodococcus yunnanensis* REPLA5, which is a strain having plastic-degrading activity, in a minimal medium supplemented with a plastic powder.
FIG. 2 shows the results of confirming the degree of corrosion (b) and the presence or absence of biofilm formation (c) after culturing *Rhodococcus yunnanensis* REPLA5 in a minimal medium supplemented with a plastic film.
FIG. 3 shows the results of analyzing the change in chemical functional groups using X-ray photoelectron spectroscopy after culturing *Rhodococcus yunnanensis* REPLA5 in a minimal medium supplemented with a plastic film.
FIG. 4 shows the results of analyzing the change in chemical functional groups using X-ray Fourier transform infrared spectroscopy after culturing *Rhodococcus yunnanensis* REPLA5 in a minimal medium supplemented with a plastic film.
FIG. 5 shows the results of analyzing the change in surface hydrophilicity using a contact angle meter after culturing *Rhodococcus yunnanensis* REPLA5 in a minimal medium supplemented with a plastic film.

### [Modes of the Invention]

One or more specific embodiments will be explained in more detail below through examples. However, these examples are for illustrative purposes of one or more embodiments, and the scope of the present invention is not limited to these examples.

### Example 1: Isolation of plastic-degrading microorganisms

### 1-1. Isolation of plastic-degrading microorganisms

A strain having plastic-degrading activity was isolated from waste polyethylene vinyl discarded in a rural soil environment.

Specifically, waste vinyl made of polyethylene was supplied to microorganisms with potential plastic-degrading ability as the sole carbon and energy source, and cultured with shaking for about 60 days under aerobic conditions at 25 °C and 180 rpm. The minimum medium composition was as follows: pH 6.51, 0.7 g NH₂PO₄, 0.7 g K₂HPO₄, 0.7 g MgSO₄·7H₂O, 1.0 g NH₄NO₃, 0.005 g NaCl, 0.002 g FeSO₄**·**7H₂O, 0.002 g ZnSO₄**·**7H₂O, and 0.001 g MnSO₄**·**H₂O, based on 1 L distilled water. After culturing, one strain that grew using polyethylene as a carbon source was isolated through a pure isolation method.

### 1.-2. Microbial identification

To identify the pure single strain obtained through the third subculture, 16S rRNA sequencing was performed, and it was confirmed that the obtained strain belonged to the genus *Rhodococcus*. The isolated microorganism was named *Rhodococcus yunnanensis* REPLA5 and deposited with the Korean Agricultural Culture Collection (KACC) on December 7, 2023, receiving accession number KACC 81286BP.

Hereinafter, the strain isolated by the present invention is referred to as "RIPLA5."

### Example 2: Verification of plastic-degrading activity

### 2-1. Evaluation of plastic-degrading activity of REPLA5

The plastic-degrading activity of REPLA5 was evaluated. Low density polyethylene (LDPE), as a plastic powder, was added to a minimal medium, and REPLA5 was inoculated and cultured with shaking for about 14 days under aerobic conditions (28 °C and 130 rpm). After 14 days, the culture solution was recovered, and the number of viable cells of REPLA5 was determined as colony-forming units (CFUs).

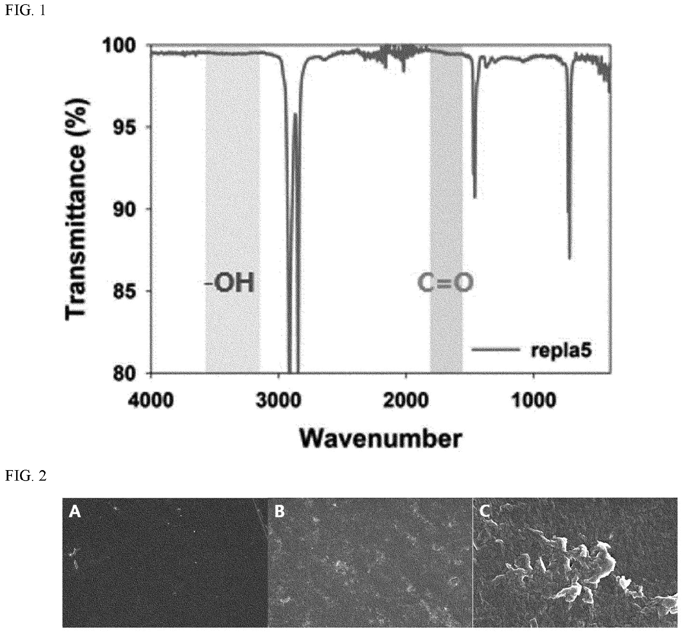

Experimental results showed a value of about 4.0 x 10^6 CFU/mL at D0, but about 7.6 x 10^7 CFU/mL at D14, and error bars were indicated as the standard deviation of the mean value (see FIG. 1). It was confirmed that REPLA5 proliferated using the plastic powder as a carbon source.

### 2-2. Evaluation of microbial growth of REPLA5

The OD600 value of the RIPLA5 culture solution cultured in Example 2-1 above was measured, and the results are shown in Table 1 below.

**[Table 1]**

| | Culture period (days) | | | | | | |
|---|---|---|---|---|---|---|---|
| | Day 1 | Day 2 | Day 3 | Day 4 | Day 5 | Day 6 | Day 7 |
| REPLA5 | 0.004 ± 0.002 | 0.000 ± 0.000 | 0.020 ± 0.004 | 0.069 ± 0.005 | 0.090 ± 0.004 | 0.103 ± 0.003 | 0.109 ± 0.005 |
| Control group | -0.001 ± 0.001 | 0.000 ± 0.001 | 0.000 ± 0.000 | 0.000 ± 0.000 | 0.000 ± 0.000 | 0.001 ± 0.001 | 0.003 ± 0.002 |

As shown in Table 1 above, no increase in optical density was observed in the control group for 7 days, whereas the optical density of REPLA5 increased due to microbial growth and reached a saturation level after about 6 days.

### 2-3. Evaluation of biofilm formation by REPLA5

To evaluate biofilm formation by REPLA5, a plastic film (LDPE) was added to a minimal medium, REPLA5 was inoculated, and the mixture was cultured with shaking for about 14 days under aerobic conditions (28 °C and 130 rpm). After 14 days, the plastic film was recovered from the culture solution, the microbial biofilm formed on the surface of the plastic film and the corrosion caused by biodegradation were observed using a scanning electron microscope, and the results are shown in FIG. 2.

As a result of the experiment, no microbial biofilm or plastic surface corrosion was observed in the control group to which REPLA5 was not added (see FIG. 2A). On the other hand, when REPLA5 was cultured for 28 days, it was confirmed that the plastic surface was corroded by biodegradation (see FIG. 2B) and that a microbial biofilm of REPLA5 was formed on the surface (see FIG. 2C).

### Example 3. Plastic degradation using REPLA5

### 3-1. Evaluation of plastic degradation rate of REPLA5

The plastic-degrading activity of REPLA5 was evaluated using a dry weight measurement method. Each plastic powder was supplied as the sole carbon source to a minimal medium, and REPLA5 was inoculated and cultured with shaking for 7 days under aerobic conditions (28 °C and 130 rpm). The plastic degradation rate of the strain was calculated according to Mathematical Formula 1, and the results are shown in Table 2. [Mathematical Formula 1] $Plastic degradation rate of REPLA 5 = \left(A-B\right) / A * 100$
(A: Initial plastic mass, B: Residual plastic mass after degradation)

**[Table 2]**

| Types of plastic | Initial mass (g) | Mass after degradation (g) | Reduced mass (g) | Degradatio n rate (%) | Standard deviation |
|---|---|---|---|---|---|
| Polyethylene (PE) | 0.2006 | 0.1945 | 0.0061 | 3.04 | 0.46 |
| Polystyrene (PS) | 0.2002 | 0.1694 | 0.0308 | 15.39 | 0.83 |
| Polypropylene (PP) | 0.2001 | 0.1901 | 0.0100 | 5.00 | 1.85 |
| Polyethylene terephthalate (PET) | 0.2007 | 0.1850 | 0.0157 | 7.82 | 1.13 |
| Polyurethane (PU) | 0.2005 | 0.1939 | 0.0066 | 3.29 | 0.31 |

As shown in Table 2 above, the plastic degradation rates (%) of REPLA5 were confirmed to be 3.04% for polyethylene, 15.39% for polystyrene, 5.00% for polypropylene, 7.82% for polyethylene terephthalate, and 3.29% for polyurethane.

Through this, it was confirmed that REPLA5 is a strain capable of degrading various types of plastics.

### 3-2. Evaluation of plastic oxidation using REPLA5

Plastics are hydrophobic materials, and in order to biologically degrade them, a process of rendering the plastics hydrophilic through oxidation should be undergone. A plastic film (LDPE) was supplied as the sole carbon source to evaluate whether REPLA5 possessed plastic-oxidizing activity.

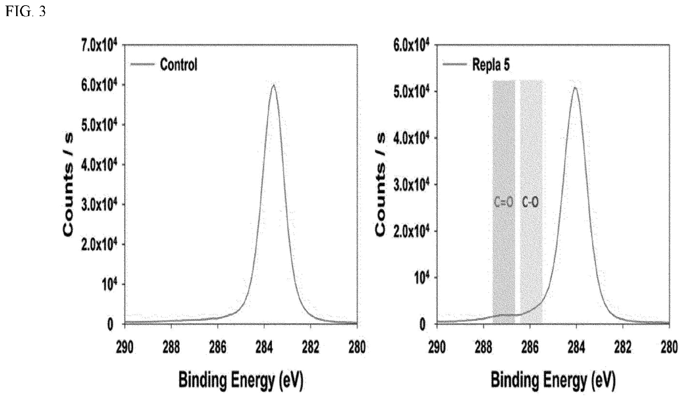

Specifically, a plastic powder was supplied to a minimal medium, and REPLA5 was inoculated and cultured with shaking for 14 days under aerobic conditions (28 °C and 130 rpm). After 14 days, LDPE was recovered from the culture solution, and the oxidation of the plastic was confirmed using X-ray photoelectron spectroscopy (XPS), Fourier-transform infrared spectroscopy (FT-IR), and a contact angle meter, and the results are shown in FIGS. 3 to 5.

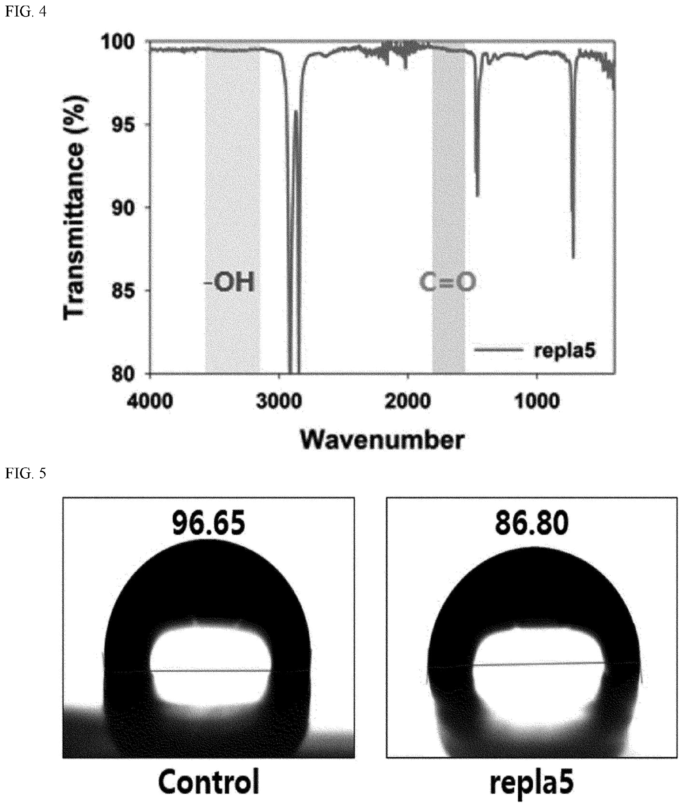

As a result of XPS analysis, it was confirmed that the oxygen element on the plastic surface increased, and a C-O bond and a carbonyl group (C=O) were formed. FT-IR analysis results showed that the hydroxyl group (OH) and the carbonyl group (C=O) were formed in the chemical functional groups on the plastic surface. Considering the appearance of the C-O bond confirmed in the XPS analysis, the hydroxyl group (OH) observed in the FT-IR analysis can be considered to be bound to the plastic in the form of C-OH (see FIG. 4). This means that REPLA5 can oxidize plastic.

In addition, through contact angle measurements, the contact angle of the plastic cultured with REPLA5 was 96.65 for the control group, but decreased to 86.80, and a contact angle less than 90 indicates hydrophilicity. In other words, it was confirmed that the hydrophilicity of the plastic increased when REPLA5 was cultured together with LDPE.

Through this evaluation of plastic oxidation, it was confirmed that the REPLA5 strain can degrade plastic when cultured alone or in combination with other strains and can also improve the degradation rate of plastic.

### [Reference Materials]

(Patent Document 1) KR 10-0350928 B1
(Patent Document 1) KR 10-0513931 B1

### [Deposit number]

Depository Institution Name: Korean Agricultural Culture Collection (KACC)
Deposit number: KACC 81286BP
Deposit Date: 20231207

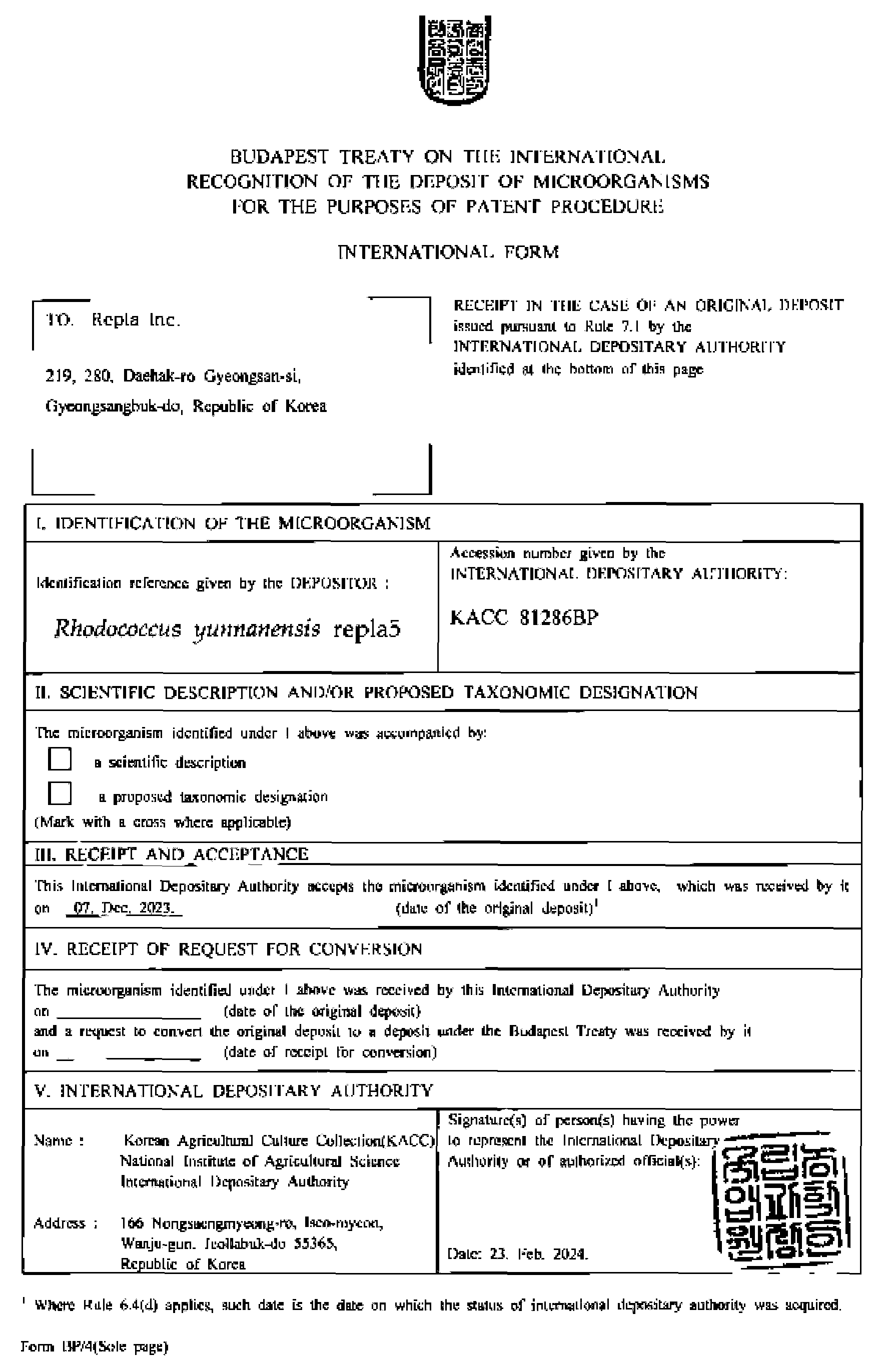

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO. Repla Inc.

219, 280, Daehak-ro Gyeongsan-si,
Gyeongsangbuk-do, Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the DEPOSITOR :

Rhodococcus yunnanensis repla5 | Accession number given by the
INTERNATIONAL DEPOSITARY AUTHORITY:

KACC 81286BP
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above, which was received by it
on 07. Dec. 2023. (date of the original deposit)¹
IV. RECEIPT OF REQUEST FOR CONVERSION
The microorganism identified under I above was received by this International Depositary Authority
on ________________ (date of the original deposit)
and a request to convert the original deposit to a deposit under the Budapest Treaty was received by it
on __ __________ (date of receipt for conversion)
V. INTERNATIONAL DEPOSITARY AUTHORITY
Name : Korean Agricultural Culture Collection(KACC)
National Institute of Agricultural Science
International Depositary Authority

Address : 166 Nongsaengmyeong-ro, Iseo-myeon,
Wanju-gun, Jeollabuk-do 55365,
Republic of Korea | Signature(s) of person(s) having the power
to represent the International Depositary
Authority or of authorized official(s):


Date: 23. Feb. 2024.

¹ Where Rule 6.4(d) applies, such date is the date on which the status of international depositary authority was acquired.

Form BP/4(Sole page)

## Claims

1. A *Rhodococcus yunnanensis* REPLA5 strain having plastic-degrading activity, deposited under accession number KACC 81286BP.

2. The *Rhodococcus yunnanensis* REPLA5 strain of claim 1, wherein the plastic is one or more selected from the group consisting of polyethylene terephthalate (PET), polyvinyl chloride (PVC), polystyrene (PS), polypropylene (PP), polyurethane (PU), and polyethylene (PE).

3. A method of degrading a plastic, comprising culturing the plastic with a *Rhodococcus yunnanensis* REPLA5 strain deposited under accession number KACC 81286BP, a culture broth of the strain, a cell lysate of the strain, or a plastic-degrading enzyme derived from the strain.

4. The method of claim 3, wherein the plastic is one or more selected from the group consisting of polyethylene terephthalate (PET), polyvinyl chloride (PVC), polystyrene (PS), polypropylene (PP), polyurethane (PU), and polyethylene (PE).

5. A composition for plastic degradation, comprising a *Rhodococcus yunnanensis* REPLA5 strain deposited under accession number KACC 81286BP, a culture broth of the strain, a cell lysate of the strain, or a plastic-degrading enzyme derived from the strain.

6. The composition of claim 5, wherein the plastic is one or more selected from the group consisting of polyethylene terephthalate (PET), polyvinyl chloride (PVC), polystyrene (PS), polypropylene (PP), polyurethane (PU), and polyethylene (PE).

7. A method of degrading a plastic metabolic intermediate, comprising culturing a plastic metabolic intermediate with a *Rhodococcus yunnanensis* REPLA5 strain deposited under accession number KACC 81286BP, a culture broth of the strain, a cell lysate of the strain, or a plastic-degrading enzyme derived from the strain.

8. The method of claim 7, wherein the plastic metabolic intermediate is one or more selected from the group consisting of an alkane, an alcohol, an aldehyde, and a fatty acid.

9. A composition for metabolic intermediate degradation, comprising a *Rhodococcus yunnanensis* REPLA5 strain deposited under accession number KACC 81286BP, a culture broth of the strain, a cell lysate of the strain, or a plastic-degrading enzyme derived from the strain.

10. A composition for promoting plastic oxidation, comprising a *Rhodococcus yunnanensis* REPLA5 strain deposited under accession number KACC 81286BP, a culture broth of the strain, a cell lysate of the strain, or a plastic-degrading enzyme derived from the strain.
